# EUROPEAN PATENT SPECIFICATION

(11) **EP 2 015 632 B1**
(45) Date of publication and mention of the grant of the patent: **02.12.2015**
(21) Application number: 07755674.4
(22) Date of filing: 19.04.2007
(51) Int. Cl.: A01N 25/02, A61K 9/12, A61F 13/00

(54) **STABLE HYDROALCOHOLIC ORAL SPRAY FORMULATIONS AND METHODS**
STABILE WÄSSRIG-ALKOHOLISCHE MUNDSPRAYFORMULIERUNGEN UND VERFAHREN
FORMULATIONS HYDROALCOOLIQUES STABLES À PULVÉRISER DANS LA CAVITÉ BUCCALE ET PROCÉDÉS ASSOCIÉS

(30) Priority: 19.04.2006 US 792942 P
(43) Date of publication of application: 21.01.2009
(73) Proprietor: Mist Pharmaceuticals, LLC, Cranford, NJ 07016 (US)
(72) Inventor: BLONDINO, Frank E., Easton, PA 18042 (US); MALITZ, Howard, Flemington, NJ 08822 (US)
(74) Representative: Teuten, Andrew John
(86) International application number: PCT/US2007/009496
(87) International publication number: WO 2007/123955

(56) References cited:
- WO-A2-2006/041843
- US-A1- 2003 095 925
- US-A1- 2003 095 925
- US-A1- 2003 129 242
- US-A1- 2003 129 242
- US-A1- 2004 042 970
- US-A1- 2006 165 606

## Description

### FIELD OF THE INVENTION

The field of the this invention is hydroalcoholic oral spray pharmaceutical formulations, methods of manufacturing such formulations, and their use for obtaining fast blood levels of the active ingredient via absorption to the systemic circulatory system through the oral mucosa in human and non-human mammals.

### BACKGROUND OF THE INVENTION

There are several limitations associated with administration of pharmaceutically active compounds through the gastrointestinal tract. The harsh environment of the gastrointestinal tract may alter the pharmaceutically active ingredient and decrease the available dosage. Metabolism by the liver may also limit the available dosage. Furthermore, patients with gastrointestinal sensitivities may have undesired side effects resulting from the gastrointestinal route of delivery. Oral sprays may provide substantial benefits compared to other modes of drug administration including faster appearance of the pharmaceutically active ingredient in the blood, improved dosage reliability, improved safety profile, and increased bioavailability.

In order to be effectively absorbed by the oral mucosa, oral transmucosal spray formulations must produce spray patterns of a suitable ovality and particle size, and be delivered in a suitable unit dose volume to ensure maximal absorption and avoid unintended administration through the gastrointestinal tract by swallowing. What is needed are stable oral spray formulations in suitable unit dose volumes which produce spray particles for rapid delivery of the active ingredient via absorption to the systemic circulatory system through the oral mucosa.

### SUMMARY OF THE INVENTION

Preferred embodiments of the invention provide stable spray formulations of an active pharmaceutical agent producing spray particles or droplets having an ovality and median diameter effective for administration to the systemic circulatory system through the oral mucosa. Preferred embodiments of the invention provide oral spray compositions comprising an active pharmaceutical agent, a solvent, and a viscosity modifying agent, wherein when a unit dose volume of about 25 to 400 mcL of the pharmaceutical composition is sprayed, the spray has a median particle size diameter of about 40 to about 100 microns.

Particularly preferred embodiments of the invention provide formulations comprising meloxicam and pharmaceutically acceptable salts thereof suitable for oral administration, and related methods of preparation and administration of meloxicam formulations. The invention provides stable, hydroalcoholic oral spray formulations in a simple, elegant format for fast onset of the active ingredient via absorption to the systemic circulatory system through the oral mucosa. In one embodiment, meloxicam is formulated in a hydroalcoholic, oral, propellant-free spray formulation at a concentration of about 0.1 to 2% w/w, more preferably 0.25 to 1%, and most preferably about 0.47% w/w. A particularly preferred hydroalcoholic meloxicam formulation embodiment comprises meloxicam, boric acid, potassium chloride, polyvinyl alcohol, ethyl alcohol, sodium hydroxide, and purified water.

Another embodiment of the invention provides a pharmaceutical composition comprising about 0.1 to 2% w/w of meloxicam, about 1 to 10 mg/g polyvinyl alcohol, and 100-300 mg/g of ethyl alcohol. Further embodiments of the invention provide hydroalcoholic, oral spray compositions comprising meloxicam, an alcohol, and a buffer.

In yet another embodiment of the invention, a pharmaceutically effective amount of meloxicam is delivered to the systemic circulatory system of a mammal via actuation of a spray pump adapted for administration of the formulations to the oral mucosal surfaces. Further embodiments of the invention provide preservative-free hydroalcoholic meloxicam formulations and methods for their preparation.

Additional embodiments of the invention provide methods of treating inflammation by administering to an animal or human subject an oral spray composition according to the invention. Preferred embodiments administer a spray volume of about 25-400 mcL, preferably about 50-200 mcL, and more preferably about 100 mcL to the oral mucosa. In another embodiment the spray volume is about 50 mcL. The volume of spray preferably contains a dose of meloxicam in the range from about 0.025 mg to about 2 mg per kg per day, preferably about 0.05 mg to about 1 mg per kg per day, and more preferably about 0.1 to 0.2 mg per kg per day.

Additional features and advantages of the invention will be set forth in the description which follows and will be apparent from the description or may be learned by practice of the invention.

### DETAILED DESCRIPTION OF THE INVENTION

Reference will now be made in detail to the presently preferred embodiments of the invention, which, together with the following examples, serve to explain the principles of the invention. It is to be understood that the application of the teachings of the present invention to a specific problem or environment will be within the capabilities of one having ordinary skill in the art in light of the teachings contained herein. Illustrative embodiments of the products of the present invention and processes for their preparation and use appear in the following examples.

Preferred embodiments of the present invention provide hydroalcoholic oral spray compositions comprising an active pharmaceutical agent, a solvent, and a viscosity modifying agent, wherein when a unit dose volume of about 25 to 400 mcl of the oral spray composition is sprayed, the spray has a median particle size diameter of about 40 to about 100 microns.

Further preferred embodiments of the present invention provide stable, essentially preservative-free pharmaceutical compositions which are hydroalcoholic solutions comprising a therapeutically effective amount of meloxicam. The preferred compositions do not resort to use of a preservative, but instead achieve inhibition of microbial growth by including an alcohol, preferably at least about 10% ethanol, in the formulation.

Meloxicam is a non-steroidal anti-inflammatory drug of the oxicam class. Chemically, meloxicam is defined as 4-hydroxy-2-methyl-N-(5-Methyl-1,3-thiazol-2-yl)-2H-1,2-benzothiazine-3-carboxamide, 1,1-dioxide. Although preferred embodiments of the invention relate to meloxicam, additional or other active pharmaceutical agents can be used in the spray delivery vehicles and methods of the invention.

The formulations according to the invention may also contain additional active pharmaceutical agents, or use such active pharmaceutical agents in place of meloxicam, such as, for example, analgesics, non-steroidal anti-inflammatory drugs (NSAIDs), corticosteroids, hyaluronan, and opioids including salts thereof. Anti-inflammatory drugs such as alosetron, anakinra, beclomethasone, betamethasone, budesonide, celecoxib, dobetasol, cromolyn, desoximetasone, dexamethasone, epinastic, etanercept, etoricoxib, flunisolide, fluocinonide, fluticasone, formoterol, hydrocortisone, hydroxychloroquine, ibudilast, ketotifen, mesalamine, methotrexate, methylprednisolone, mometasone, montelukast, nedocromil, olsalazine, prednisone, ramatroban, rofecoxib, salbutamol, salmeterol, salsalate, terbutaline, triamcinolone, valdecoxib, zafirlukast, including salts and mixtures thereof can also be included as active pharmaceutical agents in formulations of the invention.

Celecoxib is a highly selective COX-2 inhibitor which is more selective for COX-2 inhibition over COX-1. Celecoxib can reduce inflammation and pain while minimizing gastrointestinal reactions. In one embodiment, formulations of the invention may contain celecoxib. In another embodiment, methods of coadministering meloxicam and celecoxib are provided for reducing inflammation and pain, for example, before, during, or after a medical or dental procedure.

In addition, the following therapeutic classes are also suitable for inclusion in the formulations of the invention or use in place of meloxicam in formulations of the invention. Illustrative examples include analgesics such as acetaminophen; NSAIDS such as aspirin, diclofenac, etodolac, ibuprofen, indomethacin, ketoprofen, nabumetone, naproxen, piroxicam, salsalate, and sulindac, including salts thereof; corticosteroids such as betamethasone, hydrocortisone, methylprednisolone, mometasone, and triamcinolone, including salts thereof; and opioids such as codeine, hydrocodone, hydromorphone, morphine, oxycodone, and tramadol, including salts thereof.

Other suitable active pharmaceutical agents suitable for inclusion in the formulations of the invention include, but are not limited to, ondansetron, sumatriptan, zolpidem, tizanidine, ropinerole, insulin, glucose, and nitroglycerine and the active ingredients disclosed in U.S. Patent Numbers 5,869,082; 5,955,098; 6,391,282; 6,110,486; 6,676,931; 6,969,508; 6,977,070; and 6,998,110 and U.S. Patent Application Number 09/537,118, the disclosures of which are incorporated herein by reference in their entirety.

Under stability analyses, the storage stable compositions of the present invention show remarkable maintenance of the initial active agent concentration and reductions in impurities as compared to previous formulations. For example, after four months at 40°C and 75% RH, meloxicam concentration increased from 94% to 101%. Without being limited to any particular theory, the increased concentration of meloxicam is believed to be due to the evaporation of alcohol. The level of impurity B was less than 0.1% through eight weeks, 0.1 % at twelve weeks, and 0.4% at four months when stored at 40°C and 75% RH. The stability of formulations in accordance with preferred embodiments of the invention are believed to be superior to the prior art and other tested formulations as discussed below and shown, for example, in Tables 1-4.

As used herein, "storage stable" means liquid pharmaceutical formulations in which the concentration of the active ingredient is substantially maintained during storage stability testing, and degradation products and/or impurities which are typically observed in storage stability testing of such formulations are absent or significantly reduced during storage stability testing. In one embodiment, storage stability is determined at a temperature range from about 5°C to about 80°C, and more preferably from about 15°C to about 30°C. In another embodiment, storage stability is determined at a relative humidity ("RH") range from about 30% RH to about 90% RH, and more preferably from about 65% RH to about 75% RH. Preferred time intervals for measuring storage stability range from about 1 week to 2 years, more preferably from about 2 weeks to about 4 months, and most preferably at intervals of 2 weeks, 4 weeks, 8 weeks, 12 weeks, and 4 months.

Preferred formulations of the invention contain about 15% ethanol and about 0.5% of a viscosity modifying (or enhancing) agent such as polyvinyl alcohol. Without wishing to be bound by theory, it is believed that the inclusion of ethanol inhibits microbial growth in the formulation and leads to increased stability of the formulation. Other alcohols such as benzyl alcohol, chlorobutanol, glycerol, the parabens (for example, butylparaben, methylparaben), phenol, phenoxyethanol, phenylethyl alcohol, and propylene glycol, may be used in place of ethanol for this purpose. Thus, in accordance with one embodiment of the invention, it is not necessary to include an antimicrobial component or agent to ensure safe storage without the proliferation of pathogenic molds, yeasts, or bacteria. In another embodiment of the invention, various antimicrobials which are suitable for use in foods and other ingestible substances are known in the art and can be used in the present invention. Examples include the parabens (butylparaben, methylparaben, and propylparaben), propyl-p-hydroxybenzoates, sodium benzoate, and sorbic acid including salts thereof. A preferred antimicrobial agent is sodium benzoate.

Various buffers and buffer salts used to maintain pH also are suitable for use in the present invention. The formulations according to the invention will typically have a pH of about 7.0 to 12.0, more preferably a pH from about 7.5 to about 9.5, most preferably a pH of about. 8.5. Examples of buffers include ammonium hydroxide, carbonate, citrate, glycine, maleate, and phosphate, including salts thereof.

Preferred embodiments of the invention are directed to buccal spray formulations for fast onset of the active ingredient via absorption to the systemic circulatory system through the oral mucosa. Therefore, preferred spray formulations of the invention maximize absorption to the systemic circulatory system and minimize or avoid absorption by other body systems (e.g., lungs, digestive system). The size of the spray particles contributes to whether the particle is absorbed into body systems other than the oral mucosa/circulatory system (e.g., lungs). For example, smaller sized particles are more likely to be inhaled. By "buccal" herein we mean of, or pertaining to, the mouth and oral cavity, including but not limited to the oral mucosal surfaces of the tongue, cheeks, gums and/or sublingual surfaces.

In one embodiment, the percentage of the particles (droplets) of the spray formulation (e.g., after actuation of a spray pump) having a diameter of less than ten microns is less than about 10%, more preferably less than about 5%. In another embodiment, the median diameter of the spray particles is from about 20 microns to about 150 microns, more preferably from about 40 microns to about 100 microns, and most preferably about 50 microns (e.g., Tables 4 and 5).

The ovality of the spray pattern indicates whether the spray is symmetrical. It is believed that the more symmetrical the oval shape of the pattern of spray particles, the more likely the particles will evenly cover the oral mucosa. In accordance with a preferred embodiment of the invention, the ovality ratio of the pattern is between about 0.5 and about 2.0, more preferably between about 0.75 about 1.5 (e.g., Tables 5 and 6). In one embodiment, increasing the viscosity of the formulation decreases the ovality of the spray pattern. In another embodiment, an increased concentration of polyvinyl alcohol or other viscosity modifying agent decreases the ovality of the spray pattern making the spray more symmetrical.

In preparing the formulations of the present invention, the active meloxicam component or other active pharmaceutical agent is preferably incorporated into a hydroalcoholic solution. Preferably, water, borate buffer, and ethanol are used as solvents in the formulations of the invention. However, it is recognized that other solvents may be used (e.g., ammonium hydroxide buffer, carbonate, citrate, glycine, maleate, and phosphate, including salts thereof) and alcohols (for example, benzyl alcohol, glycerin, glycofurol, polyethylene glycol, propylene glycol, and sucrose) which aid in maintaining the pH of the formulation and solubilizing the active agent. Similarly, the use of polyvinyl alcohol as a viscosity modifying agent is only one possible option. Other viscosity modifying agents include such as acacia, alginic acid, bentonite, carbomer, carboxymethylcellulose, carrageenan, cellulose, ceratonia, cetyl alcohol, chitosan, colloidal silicon dioxide, ethylcellulose, gelatin, guar gum, hydroxyethyl cellulose, hydroxyethylmethyl cellulose, hydroxypropyl cellulose, hypromellose, magnesium aluminum silicate, maltitol, maltodextrin, medium-chain triglycerides, methylcellulose, polydextrose, polyethylene oxide, povidone, propylene glycol alginate, sodium alginate, stearyl alcohol, sucrose, tragacanth, trehalose, wax, and xanthan gum including salts thereof. The concentrations of these viscosity modifying agents can be adjusted to provide desired flow properties of the product by various methods, including those disclosed in <911> Viscosity, United States Pharmacopeia-National Formulary (USP 29 - NF 24) (2006), is hereby incorporated herein by reference in its entirety.

The formulations also may contain an optional propellant for delivery as an aerosol spray, or can be propellant-free and delivered by a metered valve spray pump. Suitable propellants include, but are not limited to, hydrocarbons (butane, propane, etc.), chlorofluorocarbons (CFC-11, CFC-12, etc.), hydrofluorocarbons (HFA-134a, HFA-227ea, etc.), and ethers (dimethylether, diethylether, etc.).

Optional sweetening, taste masking, or flavoring agents such as Neotame®, sorbitol, Splenda® (sucralose), sucrose, or Sunett® (acesulfamate K) also may be added if desired.

Various flavors or flavoring agents may be included to impart a pleasant taste. A pleasant taste is particularly important when the formulation is intended for administration to children or animals. Numerous flavors that are commonly used in pharmaceuticals, foods, candies, and beverages are also suitable for use in the present invention. Examples include beef, bubble gum, chicken, fish, fruit, licorice, peppermint, and other flavors. Sweeteners such as Neotame®, sorbitol, Splenda® (sucralose), sucrose, or Sunett® can also be used to adjust the flavor of the formulation.

The formulations of the present invention can be prepared by various methods. One embodiment of a manufacturing method is as follows. Preferably, meloxicam is dissolved in a strongly alkaline solution to achieve dissolution at the concentrations in the stock solution. In one embodiment, borate buffer is added prior to ethanol. Without being bound by theory, it is believed that the borate buffer protects against a decrease in apparent pH that is observed when ethanol is added. Without borate buffer, it is believed that the addition of ethanol reduces the apparent pH and the meloxicam may precipitate out of solution.

### Formula:

| **Item#** | | **mg/g** |
|---|---|---|
| 1 | Meloxicam, BP | 4.65 |
| 2 | Boric Acid, NF | 0.77 |
| 3 | Potassium Chloride, USP | 0.93 |
| 4 | Polyvinyl Alcohol, USP | 5.00 |
| 5 | Ethyl Alcohol, Dehydrated, USP | 150.00 |
| 6 | Sodium Hydroxide, NF/FCC | 1.08 |
| 7 | Purified Water, USP | 837.57 |

### Formulation Solution Concentrations

| **Item#** | | **mg/g** |
|---|---|---|
| 8 | 0.93% (w/w) Meloxicam Stock Solution | 500 |
| 9 | 5% (w/w) Polyvinyl Alcohol Solution | 100 |
| 10 | Ethyl Alcohol, Dehydrated; USP | 150 |
| 11 | 50 mM Alkaline Borate Buffer, pH 8.4 | 200 |
| 12 | Hydrochloric Acid (aq) | As needed for pH to 8.5+/-2 |
| 13 | Boric Acid Buffer | QS final weight |

### Process:

### Step

A Make 0.93% meloxicam Stock Solution:
   (a) dissolve 0.93 g of meloxicam, BP in 94 g of purified water, USP and 4.95 g of 1M sodium hydroxide solution to a 100 g solution;
   (b) completely dissolve meloxicam and determine if the pH is 11.5; if the pH is 11.5, QS to 100 g with purified water;
   (c) if the pH is not within the range of 11.5 +/- 0.2, then add 1M sodium hydroxide to adjust the pH to 11.5 +/- 0.2, QS with purified water to 100 g,
   (d) mix well.
B. Make 1 M Sodium Hydroxide as follows for 1000 mL:
   (a) transfer approximately 500 mL of Purified Water, USP;
   (b) accurately weigh 40 g of sodium hydroxide, NF ad transfer to the 1 L volumetric flask;
   (c) allow solution to cool and QS to 1 L;
   (d) mix well.
C. Make 50 mM Alkaline Borate Buffer (pH 8.4) as follows for 200 mL:
   (a) dissolve 12.37 g of boric acid, NF and 14.91 g of potassium chloride, USP in a 1000 mL volumetric flask with 750 mL of purified water, and dilute with water to achieve a volume of 1000 mL (solution C1);
   (b) make 0.2 M sodium hydroxide by accurately weighing 0.8 g of sodium hydroxide, NF and transferring the resulting solution to a 100 mL volumetric flask containing 50 mL of purified water, allow solution to cool and QS to 100ml with purified water, and mix well (solution C2);
   (c) combine 50 mL of solution C1 with 8.6 mL of solution C2 in a 200 mL volumetric flask and QS with purified water to 200 mL;
   (d) mix well.
D Make 5% Polyvinyl Alcohol as follows for 100g:
   (a) combine 5g of polyvinyl alcohol, USP with 95g of purified water, USP in an appropriate sized vessel;
   (b) stir with heat in order to dissolve the polyvinyl alcohol;
   (c) allow to cool before use;
   (d) mix well.
E Make 0.2M Hydrochloric Acid solution as follows for 25mL:
   (a) pipette 2mL of 10% Hydrochloric Acid, NF into a 25mL volumetric flask containing 15mL of purified water, USP;
   (b) dilute to volume with purified water, USP;
   (d) mix well.
F Make 0.47% Meloxicam with 15% Ethyl Alcohol as follows for 100g:
   (a) transfer 50g of 0.93% Meloxicam Stock Solution to an appropriate vessel;
   (b) add 10g of 5% Polyvinyl Alcohol Solution and mix well;
   (c) add 20g of pH 8.4 Alkaline Borate Buffer and mix well;
   (d) add 15g of Ethyl Alcohol, Dehydrated and mix well;
   (e) adjust the pH to 8.5 ± 0.2 with 0.2M Hydrochloric Acid;
   (f) dilute to weight with pH 8.4 Alkaline Borate Buffer;
   (g) mix well.

The preferred presentation of the product is in pharmaceutically acceptable glass, PET, or HDPE bottles with a capacity of between 1 and 100mL. To ensure long-term photostability, amber glass can be utilized but may not be required. Additionally, if PET or HDPE is chosen, the bottle may be opaque to ensure long-term photostability. The product is preferably dispensed using a metered pump device capable of delivering between 25 and 400 mcL. In one embodiment, the pump and actuator are modified such that a spray is dispensed horizontally to the bottle. This will allow easy dispensing to the mouth of the patient. The actuator may include an extension to allow for delivery to the buccal area of humans or animals.

The present invention also provides methods of treating various conditions in a subject (e.g., osteoarthritis, rheumatoid arthritis, inflammation, gout, and pain). The methods include administering to a subject in need of treatment a pharmaceutical composition according to the invention. In one embodiment, the subject is a human; in another embodiment the subject is a non-human mammal, preferably selected from the group of dogs, cats, horses, cattle, sheep, and swine. The preferred storage stable meloxicam compositions can be administered to a patient in any suitable dosage range, for example, 0.025 mg to about 2 mg per kg per day, preferably about 0.05 mg to about 1 mg per kg per day, and more preferably 0.1 to 0.2 mg per kg per day.

It is to be understood that application of the teachings of the present invention to a specific problem or environment will be within the capability of one having ordinary skill in the art in light of the teachings contained herein. The present invention is more fully illustrated by the following non-limiting examples.

### EXAMPLE 1

Four formulations were prepared for stability testing. Formula 1 is 0.47% Meloxicam with 0.5% Glycofurol (Table 1), Formula 2 is 0.47% Meloxicam with 0.5% PVA and 7.5% EtOH (Table 2), Formula 3 is 0.47% Meloxicam with 0.5% PVA and 15% EtOH (Table 3), and Formula 4 is 0.47% Meloxicam with 0.5% PVA and 15% EtOH in Borate Buffer (Table 4). The formulas were stored at 25°C/60% RH, 30°C/65% RH, 40°C/75% RH, and 5°C. The formulations stored at 25°C/60% RH and 40°C/75% RH were tested at the following intervals: 2 weeks, 4 weeks, 8 weeks (2 months), 12 weeks, and 4 months.

In addition to these conditions, a cycling study was performed where all three formulations were kept at 5°C each night from approximately 4 pm to 8 am the next morning. The formulations were stored at 40°C/75% RH for the duration of the working day, approximately 8 hours. The formulations were not cycled on the weekends or holidays. The stability of the formulations was tested at 4 months after initiation of the cycling study.

The initial Meloxicam concentration in Formula 1 was 96.8% and dropped to 82.2% at the 4 month 40°C/75% RH time points. In addition, an Impurity B was identified from analytical testing. The impurity appeared at a concentration 0.1% at 2 weeks and increased to 0.7% at 4 months.

The initial Meloxicam concentration in Formula 2 was 95.0%. The concentration increased to 96.4% after 4 months at 40°C/75% RH. At 40°C/75% RH, the concentration of Impurity B was less than 0.1% through 8 weeks. The concentration of Impurity B was 0.2% at 12 weeks and 0.5% at 4 months. The level of Impurity B was lower in Formula 2 then in Formula 1.

The initial Meloxicam concentration was 94.0% in Formula 3 and increased to 100.8% after 4 months at 40°C/75% RH. The increase in concentration may be due to the concentrating of the formulation by evaporation of alcohol. At 40°C/75% RH, Impurity B was less than 0.1% through 8 weeks. The concentration of Impurity B was 0.1% at 12 weeks and 0.4% at 4 months. The concentration of Impurity B is Formula 3 was lower than that observed for Formulas 1 and 2.

The initial Meloxicam concentration was 100.5% in Formula 4 and remained unchanged after 2 months at 25°C/65% RH and 40°C/75% RH. At 40°C/75% RH, Impurity A was less than 0.05% through 2 months. The concentration of Impurity B is Formula 4 was lower than that observed for Formulas 1, 2, and 3. Overall degradation in Formula 4 was similar to Formula 3.

Two formulas were prepared for a spray study (Tables 5-6) both containing 0.47% Meloxicam and 15% EtOH. Formula 5 has a concentration of 0.5% PVA and Formula 6 has a concentration of 0.25% PVA. The following four characteristics of the formulas were observed: (1) percentage of particles that have a diameter less than 10µm, (2) cumulative distribution Dv(50), (3) cumulative distribution Dv(90), and (4) ovality. The percentage of the diameter of particles less than 10 µm may indicate the percentage of the particles at risk of being inhaled into the lung. The higher the Dv value, the fewer particles that can be inhaled. For the formulations submitted, the amount of particles less than 10 µm is 1.6% for the Formula 5 and 2.4% for Formula 6.

The Dv (50) and Dv (90) values are size values corresponding to the cumulative distribution of particles at 50% and 90%. Thus, the Dv (90) represents a size below which 90% of the cumulative distribution occurs. From this it can be inferred that the Dv (50) value corresponds to the median diameter. The Dv (50) and Dv (90) for both formulas are very similar.

The ovality is defined as the ratio of Dₘₐₓ and Dₘᵢₙ. Dmax is defined as the largest chord, in mm, that can be drawn within the spray pattern that crosses the COMw (i.e., center of mass of the spray pattern) in base units. Dₘᵢₙ is described as the smallest chord, in mm, that can be drawn within the spray pattern that crosses the COMw in base units. COMw is defined as the center of mass of the detected spray pattern, where each pixel's intensity is taken into account. With regard to Formulas 5 and 6, the closer the ovality is to 1.0, the more symmetrical the shape of the spray. Formulation 5 has an ovality of 1.26 and Formula 6 has an ovality of 1.51. The 0.5% PVA formulation has a more preferred ovality.

**Table 1. Stability of Meloxicam 0.47% with Glycofurol**

| Stability Condition | Spray Weight | Spray Content | SC/SW Ratio | % Label Claim | Impurity B&C | Other Impurity |
|---|---|---|---|---|---|---|
| Initial | N/A | Bulk 0.484% RSD: 0.2% | N/A | 96.80% | N/D | Unknown 0.7% |
| 25/60/2wk, n=3 | 98.4mg RSD:1.5% | 0.461mg, RSD: 1.1% | 0.00468 RSD: 0.4% | 92.10% | <0.10% | N/D |
| 25/60/4wk, n=3 | 100.2mg RSD:2.5% | 0.475mg RSD: 2.3% | 0.00474 RSD: 0.5% | 95.00% | Imp. B: 0.18% | N/D |
| 25/60/8wk, n=3 | 91.6mg RSD:9.4% | 0.427mg RSD: 9.0% | 0.00466 RSD: 0.5% | 85.40% | Imp. B: 0.14% | N/D |
| 25/60/12wk, n=3 | 99.8mg RSD:1.0% | 0.467mg RSD: 1.3% | 0.00468 RSD: 1.0% | 93.50% | Imp. B: 0.20% | N/D |
| 25/60/4mo, n=3 | 100.7mg RSD:0.4% | 0.473mg RSD: 1.1% | 0.00469 RSD: 0.9% | 94.50% | Imp. B: 0.27% | N/D |
| 25/60/4mo, cycle n=3 | 90.5mg RSD:15.1% | 0.418mg RSD: 14.9% | 0.00462 RSD: 0.3% | 83.60% | Imp. B: 0.25% | N/D |
| 40/75/2wk, n=3 | 96.1mg RSD:2.5% | 0.450mg RSD: 2.4% | 0.00468 RSD: 0.3% | 89.90% | Imp. B: 0.10% | N/D |
| 40/75/4wk, n=3 | 86.0mg RSD:12.8% | 0.411mg RSD: 13.0% | 0.00478 RSD: 0.3% | 82.10% | Imp. B: 029% | N/D |
| 40/75/8wk, n=3 | 84.9mg RSD:10.1% | 0.396mg RSD: 9.8% | 0.00466 RSD: 0.4% | 79.20% | Imp. B: 0.33% | N/D |
| 40/75/12wk, n=3 | 86.0mg RSD:9.5% | 0.402mg RSD: 9.4% | 0.00467 RSD: 0.4% | 80.40% | Imp. B: 0.49% | N/D |
| 40/75/4mo, n=3 | 85.4mg RSD:8.0% | 0.411mg RSD: 8.1% | 0.00481 RSD: 2.0% | 82.20% | Imp. B: 0.67% | N/D |

**Table 2. Stability of Meloxicam 0.47% with 0.5% PVA and 7.5% EtOH**

| Stability Condition | Spray Weight | Spray Content | SC/SW Ratio | % Label Claim | Impurity B&C | Other Impurity |
|---|---|---|---|---|---|---|
| Initial | N/A | Bulk 0.475% RSD: 0.4% | N/A | 95.00% | N/D | Unknown 0.5% |
| 25/60/2wk, n=3 | 98.4mg RSD.0.5% | 0.467mg RSD: 0.7% | 0.00475 RSD: 0.3% | 93.40% | <0.10% | N/D |
| 25/60/4wk, n=3 | 98.6mg RSD:1.5% | 0.481mg RSD: 2.0% | 0.00488 RSD: 0.5% | 96.20% | <0.10% | N/D |
| 25/60/8wk, n=3 | 90.3mg RSD:11.5% | 0.436mg RSD: 11.9% | 0.00483 RSD: 0.5% | 87.20% | <0.10% | N/D |
| 25/60/12wk, n=3 | 90.5mg RSD:11.3% | 0.430mg RSD: 11.5% | 0.00475 RSD: 0.4% | 85.90% | <0.10% | N/D |
| 25/60/4mo, n=3 | 96.7mg RSD:2.4% | 0.463mg RSD: 3.6% | 0.00479 RSD: 1.8% | 92.70% | <0.10% | N/D |
| 25/60/4mo, cycle n=3 | 98.7mg RSD:5.4% | 0.466mg RSD: 5.0% | 0.00472 RSD: 0.4% | 93.10% | <0.10% | N/D |
| 40/75/2wk, n=3 | 99.1mg RSD:0.6% | 0.476mg RSD: 0.3% | 0.00480 RSD: 0.4% | 95.30% | <0.10% | N/D |
| 40/75/4wk, n=3 | 92.8mg RSD:11.9% | 0.456mg RSD: 12.0% | 0.00491 RSD: 0.9% | 91.20% | <0.10% | N/D |
| 40/75/8wk, n=3 | 96.6mg RSD:2.7% | 0.462mg RSD: 2.3% | 0.00478 RSD: 0.9% | 92.50% | <0.10% | N/D |
| 40/75/12wk, n=3 | 97.3mg RSD:1.2% | 0.464mg RSD: 2.0% | 0.00477 RSD: 1.1% | 92.80% | Imp. B: 0.18% | N/D |
| 40/75/4mo, n=3 | 98.1mg RSD:1.7% | 0.482mg RSD: 1.6% | 0.00491 RSD: 0.6% | 96.40% | Imp. B: 0.54% | N/D |

**Table 3. Stability of Meloxicam 0.47% with 0.5% PVA and 15% EtOH**

| Stability Condition | Spray Weight | Spray Content | SC/SW Ratio | % Label Claim | Impurity B&C | Other Impurity |
|---|---|---|---|---|---|---|
| Initial | N/A | Bulk 0.470% RSD: 0.4% | N/A | 94.00% | N/D | Unknown 0.5% |
| 25/60/2wk, n=3 | 98.5mg RSD:1.4% | 0.474mg RSD: 1.5% | 0.00481 RSD: 0.2% | 94.70% | <0.10% | N/D |
| 25/60/4wk, n=3 | 98.5mg RSD:1.2% | 0.485mg RSD: 1.5% | 0.00492 RSD: 0.3% | 97.00% | <0.10% | N/D |
| 25/60/8wk, n=3 | 93.7mg RSD:8.4% | 0.454mg RSD: 7.7% | 0.00485 RSD: 0.7% | 90.90% | Imp. B: 0.14% | N/D |
| 25/60/12wk, n=3 | 98.5mg RSD:1.9% | 0.481mg RSD: 1.1% | 0.00488 RSD: 0.8% | 96.20% | <0.10% | N/D |
| 25/60/4mo, n=3 | 98.8mg RSD:1.2% | 0.493mg RSD: 2.6% | 0.00499 RSD: 3.8% | 98.50% | <0.10% | N/D |
| 25/60/4mo, cycle n=3 | 98.1mg RSD:1.9% | 0.467mg RSD: 1.9% | 0.00476 RSD: 1.0% | 93.40% | <0.10% | N/D |
| 40/75/2wk, n=3 | 95.2mg RSD:4.6% | 0.459mg RSD: 4.4% | 0.00482 RSD: 0.2% | 91.80% | <0.10% | N/D |
| 40/75/4wk, n=3 | 98.5mg RSD:1.1% | 0.490mg RSD: 1.2% | 0.00497 RSD: 0.2% | 97.90% | <0.10% | N/D |
| 40/75/8wk, n=3 | 96.6mg RSD:2.7% | 0.468mg RSD: 3.3% | 0.00484 RSD: 1.4% | 93.70% | <0.10% | N/D |
| 40/75/12wk, n=3 | 97.4mg RSD:3.0% | 0.469mg RSD: 3.2% | 0.00482 RSD: 0.9% | 93.80% | Imp. B: 0.12% | N/D |
| 40/75/4mo, n=3 | 100.5mg RSD:0.6% | 0.504mg RSD: 2.9% | 0.00501 RSD: 2.9% | 100.80% | Imp.B: 0.35% | N/D |

**Table 4. Stability of Meloxicam 0.47% with 0.5% PVA and 15% EtOH in Borate Buffer**

| Test | T0 | 4 week 25ºC/60%RH | 2 month 25ºC/60%RH | 2 week 40ºC/75%RH | 4 week 40ºC/75%RH | 2 month 40ºC/75%RH |
|---|---|---|---|---|---|---|
| Assay of Formulation Conc. (n=3) | 0.473% (w/w) 100.5% LC %RSD: 0.05 | 0.467% (w/w) 99.3% LC %RSD: 0.77 | 0.473% (w/w) 100.5% LC %RSD: 0.45 | 0.473% (w/w) 100.7% LC %RSD: 0.17 | 0.469% (w/w) 99.8% LC %RSD: 0.03 | 0.472% (w/w) 100.4% LC %RSD: 0.13 |
| Impurity/ Degradent | Unknown: 0.11% | Unknown: 0.09% | Unknown: 0.06% Imp. A: 0.04% | Unknown: 0.11% | Unknown: 0.08% | Unknown: 0.10% Imp. A: 0.05% |
| pH | 7.94 | N/A | 7.79 | N/A | N/A | 7.25 |
| Spray Weight Uniformity (n=10) | 97.0mg %RSD: 0.94 | 97.5mg %RSD: 0.77 | N/A | 97.2mg %RSD: 2.81 | 96.8mg %RSD: 3.58 | N/A |
| Spray Content Uniformity (n=10) | 0.459mg 97.6% LC %RSD: 0.85 | 0.463mg 98.5% LC %RSD: 0.54 | N/A | 0.466mg 99.3% LC %RSD: 2.71 | 0.457mg 97.3% LC %RSD: 3.36 | N/A |
| Impurity/ Degradent | Unknown: 0.12% | Unknown: 0.09% | N/A | Unknown: 0.11% | Unknown: 0.10% | N/A |
| Droplet Size Distribution : | | | | | | |
| | 0.97% | 0.83% | N/A | 0.71% | 0.72% | N/A |
| %, <10 µm: | 32.07 | 34.98 | N/A | 51.75 | 45.18 | N/A |
| | 88.79 | 100.45 | N/A | 130.70 | 122.32 | N/A |
| | 141.02 | 150.59 | N/A | 182.63 | 171.31 | N/A |
| D10: | 1.23 | 1.15 | N/A | 1.00 | 1.03 | N/A |
| D50: | | | | | | |
| D90: | | | | | | |
| Spam: | | | | | | |

**Table 5. Spray Characterization Study of Meloxicam 0.47% with 0.5% PVA and 15% EtOH**

| Sample ID | <10µmm | Dv(10) | Dv(50) | Dv(90) | Span | Spray Angle | Ovality |
|---|---|---|---|---|---|---|---|
| 11 | 1.59% | 23.09 | 45.53 | 102.73 | 1.75 | 27.9 | 1.343 |
| 12 | 1.59% | 22.96 | 46.50 | 106.95 | 1.81 | 39.0 | 1.154 |
| 21 | 1.64% | 23.18 | 49.14 | 108.73 | 1.74 | 42.5 | 1.211 |
| 22 | 1.69% | 22.86 | 48.16 | 108.63 | 1.78 | 29.9 | 1.205 |
| 31 | 1.52% | 23.45 | 47.92 | 104.45 | 1.69 | 31.2 | 1.186 |
| 32 | 1.57% | 23.02 | 48.02 | 103.92 | 1.68 | 40.5 | 1.468 |
| AVERAGE | 1.60% | 23.09 | 47.55 | 105.90 | 1.74 | 35.17 | 1.26 |
| STD. DEV | 0.06% | 0.21 | 1.30 | 2.55 | 0.05 | 6.22 | 0.12 |
| RSD | 3.7 | 0.9 | 2.7 | 2.4 | 2.9 | 17.7 | 9.5 |

**Table 6. Spray Characterization Study of Meloxicam 0.47% with 0.25% PVA and 15% EtOH**

| Sample ID | <10µmm | Dv(10) | Dv(50) | Dv(90) | Span | Spray Angle | Ovality |
|---|---|---|---|---|---|---|---|
| 11 | 2.62% | 18.70 | 39.08 | 100.01 | 2.08 | 29.1 | 1.298 |
| 12 | 2.46% | 19.38 | 39.41 | 96.70 | 1.96 | 29.6 | 1.426 |
| 21 | 2.36% | 19.36 | 40.27 | 102.34 | 2.06 | 37.9 | 1.336 |
| 22 | 2.33% | 19.58 | 39.98 | 96.13 | 1.91 | 44.0 | 1.818 |
| 31 | 2.34% | 19.62 | 41.67 | 99.86 | 1.93 | 29.2 | 1.593 |
| 32 | 2.41% | 19.22 | 40.80 | 98.49 | 1.94 | 31.2 | 1.611 |
| AVERAGE | 2.42% | 19.31 | 40.20 | 98.92 | 1.98 | 33.50 | 1.51 |
| STD. DEV | 0.11% | 0.33 | 0.94 | 2.31 | 0.07 | 6.13 | 0.20 |
| RSD | 4.5 | 1.7 | 2.3 | 2.3 | 3.6 | 18.3 | 13.0 |

The above description and examples are only illustrative of preferred embodiments which achieve one or more objects, features, and advantages of the present invention, and it is not intended that the present invention be limited thereto.

## Claims

1. A hydroalcoholic spray composition, comprising an active pharmaceutical agent, a solvent, and a viscosity modifying agent, wherein when a unit dose volume of 25 to 400 microliters of the composition is sprayed, the sprayed composition has a median particle size diameter of 20 to 100 micrometers.

2. A composition according to claim 1, wherein the active pharmaceutical agent is selected from the group consisting of meloxicam, celecoxib, ondansetron, sumatriptan, zolpidcm, tizanidine, ropinerole, insulin, glucose, and nitroglycerine.

3. A composition according to claim 1, wherein the active pharmaceutical agent is meloxicam, and the composition further comprises an alcohol.

4. A composition according to claim 3, wherein said composition is propellant-free.

5. A composition according to claim 3, wherein said composition further comprises a propellant.

6. A composition according to claim 5, wherein the propellant is selected from the group consisting of hydrocarbons, chlorofluorocarbons, hydrofluorocarbons, and ethers.

7. A composition according to claim 3, wherein the concentration of meloxicam is 0.1 to 2%, w/w.

8. A composition according to claim 7, wherein the concentration of meloxicam is 0.25 to 1% w/w.

9. A composition according to claim 8, wherein the concentration of meloxicam is 0.47% w/w.

10. A composition according to claim 3, wherein said composition is essentially preservative-free.

11. A composition according to claim 3, wherein the concentration of ethyl alcohol is 15% and said viscosity modifying agent is polyvinyl alcohol at 0.5%.

12. A composition according to claim 3, wherein said composition further comprises a buffer.

13. A composition according to claim 12, wherein said buffer is selected from the group consisting of ammonium hydroxide, carbonate, citrate, glycine, maleate, phosphates and salts thereof.

14. A composition according to claim 3, wherein said composition has a pH from 7 to 12.

15. A composition according to claim 14, wherein said composition has a pH from 7.5 to 9.5.

16. A composition according to claim 15, wherein said composition has a pH of 8.5.

17. A composition according to claim 3, wherein said composition is storage stable.

18. A composition according to claim 3, wherein the percentage of particles less than 10 micrometers in diameter in the spray is 10%.

19. A composition according to claim 18, wherein the percentage of particles less than 10 micrometers in diameter in the spray is 5%.

20. A composition according to claim 3, wherein the median diameter of particles in the spray is from 20 to 150 micrometers.

21. A composition according to claim 20, wherein the median diameter of particles in the spray is from 40 to 100 micrometers.

22. A composition according to claim 21, wherein the median diameter of particles in the spray is 50 micrometers.

23. A composition according to claim 3, wherein said composition further comprises an additional active ingredient selected from the group consisting of analgesics, non-steroidal anti-inflammatory drugs, corticosteroids, hyaluronan, and opoids including salts thereof.

24. A composition according to claim 23, wherein the anti-inflammatory drug is selected from the group consisting of alosetron, anakinra, beclomethasone, betamethasone, budesonide, celecoxib, clobetasol, cromolyn, desoximetasone, dexamethasone, epinastic, etanercept, etoricoxib, flunisolide, fluocinonide, fluticasone, formoterol, hydrocortisone, hydroxychloroquine, ibudilast, ketotifen, mesalamine, methotrexate, methylprednisolone, mometasone, montelukast, nedocromil, olsalazine, prednisone, ramatroban, rofecoxib, salbutamol, salmeterol, salsalate, terbutaline, triamcinolone, valdecoxib, zafirlukast, including salts thereof.

25. A composition according to claim 3, comprising 0.1 to 2% w/w of meloxicam, 1 to 10 mg/g polyvinyl alcohol, and 100-300 mg/g of ethyl alcohol.

26. A composition for use in treating a condition in a human or a non-human animal, wherein a unit does volume of 25 microliters to 400 microliters of the pharmaceutical composition is sprayed on the oral mucosa of the animal, wherein the sprayed composition has a median particle size diameter of 40 micrometers to 100 micrometers, and wherein the active pharmaceutical agent is absorbed through the oral mucosa of the animal to provide a therapeutically effective amount of the active pharmaceutical agent to the systemic circulatory system to alleviate said condition.

27. A composition for use according to claim 26, wherein the composition comprises meloxicam, an alcohol, and a buffer.

28. A composition for use according to claim 26, wherein the active pharmaceutical agent is selected from the group consisting of meloxicam, celecoxib, ondansetron, sumatriptan, zolpidem, tizanidine, ropinerole, insulin, glucose, and nitroglycerine.

29. A composition for use according to claim 27, wherein the amount of meloxicam is from 0.1 to 2% w/w.

30. A composition for use according to claim 29, wherein the amount of meloxicam is from 0.25 to 1% w/w.

31. A composition for use according to claim 30, wherein the amount of meloxicam is 0.47% w/w.

32. A composition for use according to claim 26, wherein the spray volume is 50 to 400 microliters.

33. A composition for use according to claim 32, wherein the spray volume is 50 to 200 microliters.

34. A composition for use according to claim 33, wherein the spray volume is 100 microliters.

35. A composition for use according to claim 33, wherein the spray volume is 50 microliters.

36. A composition for use according to claim 32, wherein the spray volume is 200 microliters.

37. A composition for use according to claim 26, wherein the meloxicam is administered in a dose from 0.025 milligrams to 2 milligrams per kilogram per day.

38. A composition for use according to claim 37, wherein the meloxicam is administered in a dose from 0.05 milligrams to 1 milligrams per kilogram per day.

39. A composition for use according to claim 38, wherein the meloxicam is administered in a dose from 0.1 milligrams to 0.2 milligrams per kilogram per day.

40. A composition for use according to claim 27, wherein the condition is selected from the group consisting of osteoarthritis, rheumatoid arthritis, inflammation, gout, and pain, and the composition comprises 0.1 to 2% w/w of meloxicam, 1 to 10 mg/g polyvinyl alcohol, amd 100-300 mg/g of ethyl alcohol.

## Patentansprüche

1. Hydroalkoholische Sprühzusammensetzung, umfassend einen pharmazeutischen Wirkstoff, ein Lösungsmittel und ein viskositätsmodifizierendes Mittel, wobei, wenn man ein Einheitsdosisvolumen von 25 bis 400 Mikrolitern der Zusammensetzung sprüht, die gesprühte Zusammensetzung einen medianen Teilchengrößendurchmesser von 20 bis 100 Mikrometer aufweist.

2. Zusammensetzung nach Anspruch 1, wobei der pharmazeutische Wirkstoff aus der aus Meloxicam, Celecoxib, Ondansetron, Sumatriptan, Zolpidem, Tizanidin, Ropinerol, Insulin, Glukose und Nitroglycerin bestehenden Gruppe ausgewählt ist.

3. Zusammensetzung nach Anspruch 1, wobei es sich bei dem pharmazeutischen Wirkstoff um Meloxicam handelt und die Zusammensetzung weiterhin einen Alkohol umfasst.

4. Zusammensetzung nach Anspruch 3, wobei die Zusammensetzung treibmittelfrei ist.

5. Zusammensetzung nach Anspruch 3, wobei die Zusammensetzung weiterhin ein Treibmittel umfasst.

6. Zusammensetzung nach Anspruch 5, wobei das Treibmittel aus der aus Kohlenwasserstoffen, Fluorchlorkohlenwasserstoffen, Fluorkohlenwasserstoffen und Ethern bestehenden Gruppe ausgewählt ist.

7. Zusammensetzung nach Anspruch 3, wobei die Konzentration von Meloxicam 0,1 bis 2 Gew.-% beträgt.

8. Zusammensetzung nach Anspruch 7, wobei die Konzentration an Meloxicam 0,25 bis 1 Gew.-% beträgt.

9. Zusammensetzung nach Anspruch 8, wobei die Konzentration an Meloxicam 0,47 Gew.-% beträgt.

10. Zusammensetzung nach Anspruch 3, wobei die Zusammensetzung im Wesentlichen konservierungsstofffrei ist.

11. Zusammensetzung nach Anspruch 3, wobei die Konzentration an Ethylalkohol 15% beträgt und es sich bei dem viskositätsmodifizierenden Mittel um Polyvinylalkohol in einer Konzentration von 0,5% handelt.

12. Zusammensetzung nach Anspruch 3, wobei die Zusammensetzung weiterhin einen Puffer umfasst.

13. Zusammensetzung nach Anspruch 12, wobei der Puffer aus der aus Ammoniumhydroxid, Carbonat, Citrat, Glycin, Maleat, Phosphaten und Salzen davon bestehenden Gruppe ausgewählt ist.

14. Zusammensetzung nach Anspruch 3, wobei die Zusammensetzung einen pH-Wert von 7 bis 12 aufweist.

15. Zusammensetzung nach Anspruch 14, wobei die Zusammensetzung einen pH-Wert von 7,5 bis 9,5 aufweist.

16. Zusammensetzung nach Anspruch 15, wobei die Zusammensetzung einen pH-Wert von 8,5 aufweist.

17. Zusammensetzung nach Anspruch 3, wobei die Zusammensetzung lagerungsstabil ist.

18. Zusammensetzung nach Anspruch 3, wobei der Prozentsatz von Teilchen mit einem Durchmesser von weniger als 10 Mikrometer im Spray 10% beträgt.

19. Zusammensetzung nach Anspruch 18, wobei der Prozentsatz von Teilchen mit einem Durchmesser von weniger als 10 Mikrometer im Spray 5% beträgt.

20. Zusammensetzung nach Anspruch 3, wobei der mediane Teilchendurchmesser im Spray 20 bis 150 Mikrometer beträgt.

21. Zusammensetzung nach Anspruch 20, wobei der mediane Teilchendurchmesser im Spray 40 bis 100 Mikrometer beträgt.

22. Zusammensetzung nach Anspruch 21, wobei der mediane Teilchendurchmesser im Spray 50 Mikrometer beträgt.

23. Zusammensetzung nach Anspruch 3, wobei die Zusammensetzung weiterhin einen zusätzlichen, aus der aus Analgetika, nichtsteoridalen entzündungshemmenden Mitteln, Kortikosteroiden, Hyaluronan und Opioiden einschließlich Salzen davon bestehenden Gruppe ausgewählten Wirkstoff umfasst.

24. Zusammensetzung nach Anspruch 23, wobei das entzündungshemmende Mittel aus der aus Alosetron, Anakinra, Beclomethason, Betamethason, Budesonid, Celecoxib, Clobetasol, Cromolyn, Desoximetason, Dexamethason, Epinastic, Etanercept, Etoricoxib, Flunisolid, Fluocinonid, Fluticason, Formoterol, Hydrocortison, Hydroxychloroquin, Ibudilast, Ketotifen, Mesalamin, Methotrexat, Methylprednisolon, Mometason, Montelukast, Nedocromil, Olsalazin, Prednison, Ramatroban, Rofecoxib, Salbutamol, Salmeterol, Salsalat, Terbutalin, Triamcinolon, Valdecoxib, Zafirlukast einschließlich Salzen davon bestehenden Gruppe ausgewählt ist.

25. Zusammensetzung nach Anspruch 3, umfassend 0,1 bis 2 Gew.-% Meloxicam, 1 bis 10 mg/g Polyvinylalkohol und 100-300 mg/g Ethylalkohol.

26. Zusammensetzung zur Verwendung bei der Behandlung eines Leidens bei einem Menschen oder einem nichtmenschlichen Tier, bei dem man ein Einheitsdosisvolumen von 25 Mikrolitern bis 400 Mikrolitern der pharmazeutischen Zusammensetzung auf die Mundschleimhaut des Tieres sprüht, wobei die gesprühte Zusammensetzung einen medianen Teilchengrößendurchmesser von 40 Mikrometern bis 100 Mikrometern aufweist und wobei der pharmazeutische Wirkstoff über die Mundschleimhaut des Tieres resorbiert wird, unter Bereitstellung einer therapeutisch wirksamen Menge des pharmazeutischen Wirkstoffs an den systemischen Kreislauf zur Linderung des Leidens.

27. Zusammensetzung zur Verwendung nach Anspruch 26, wobei die Zusammensetzung Meloxicam, einen Alkohol und einen Puffer umfasst.

28. Zusammensetzung zur Verwendung nach Anspruch 26, wobei der pharmazeutische Wirkstoff aus der aus Meloxicam, Celecoxib, Ondansetron, Sumatriptan, Zolpidem, Tizanidin, Ropinerol, Insulin, Glukose und Nitroglycerin bestehenden Gruppe ausgewählt ist.

29. Zusammensetzung zur Verwendung nach Anspruch 27, wobei die Menge an Meloxicam 0,1 bis 2 Gew.-% beträgt.

30. Zusammensetzung zur Verwendung nach Anspruch 29, wobei die Menge an Meloxicam 0,25 bis 1 Gew.-% beträgt.

31. Zusammensetzung zur Verwendung nach Anspruch 30, wobei die Menge an Meloxicam 0,47 Gew.-% beträgt.

32. Zusammensetzung zur Verwendung nach Anspruch 26, wobei das Sprühvolumen 50 bis 400 Mikroliter beträgt.

33. Zusammensetzung zur Verwendung nach Anspruch 32, wobei das Sprühvolumen 50 bis 200 Mikroliter beträgt.

34. Zusammensetzung zur Verwendung nach Anspruch 33, wobei das Sprühvolumen 100 Mikroliter beträgt.

35. Zusammensetzung zur Verwendung nach Anspruch 33, wobei das Sprühvolumen 50 Mikroliter beträgt.

36. Zusammensetzung zur Verwendung nach Anspruch 32, wobei das Sprühvolumen 200 Mikroliter beträgt.

37. Zusammensetzung zur Verwendung nach Anspruch 26, wobei das Meloxicam in einer Dosis von 0,025 Milligramm bis 2 Milligramm pro Kilogramm pro Tag verabreicht wird.

38. Zusammensetzung zur Verwendung nach Anspruch 37, wobei das Meloxicam in einer Dosis von 0,05 Milligramm bis 1 Milligramm pro Kilogramm pro Tag verabreicht wird.

39. Zusammensetzung zur Verwendung nach Anspruch 38, wobei das Meloxicam in einer Dosis von 0,1 Milligramm bis 0,2 Milligramm pro Kilogramm pro Tag verabreicht wird.

40. Zusammensetzung zur Verwendung nach Anspruch 27, wobei das Leiden aus der aus Osteoarthritis, rheumatoider Arthritis, Entzündung, Gicht und Schmerzen bestehenden Gruppe ausgewählt ist und die Zusammensetzung 0,1 bis 2 Gew.-% Meloxicam, 1 bis 10 mg/g Polyvinylalkohol und 100-300 mg/g Ethylalkohol umfasst.

## Revendications

1. Composition hydroalcoolique pour pulvérisation, comprenant un agent pharmaceutique actif, un solvant et un agent rhéologique, où lorsqu'un volume de dose unitaire compris entre 25 et 400 microlitres de la composition est pulvérisé, la composition pulvérisée présente une granulométrie médiane comprise entre 20 et 100 micromètres.

2. Composition selon la revendication 1, où l'agent pharmaceutique actif est choisi dans le groupe constitué par les suivants : méloxicam, célécoxib, ondansétron, sumatriptan, zolpidem, tizanidine, ropinérole, insuline, glucose et nitroglycérine.

3. Composition selon la revendication 1, où l'agent pharmaceutique actif est le méloxicam, et la composition comprend en outre un alcool.

4. Composition selon la revendication 3, où ladite composition ne contient pas de gaz propulseur.

5. Composition selon la revendication 3, où ladite composition contient en outre un gaz propulseur.

6. Composition selon la revendication 5, où le gaz propulseur est choisi dans le groupe constitué par les hydrocarbures, les chlorofluorocarbures, les hydrofluorocarbures et les éthers.

7. Composition selon la revendication 3, où la concentration en méloxicam est comprise entre 0,1 et 2 % en masse.

8. Composition selon la revendication 7, où la concentration en méloxicam est comprise entre 0,25 et 1 % en masse.

9. Composition selon la revendication 8, où la concentration en méloxicam est de 0,47 % en masse.

10. Composition selon la revendication 3, où ladite composition ne contient essentiellement pas de conservateur.

11. Composition selon la revendication 3, où la concentration en éthanol est de 15 % et ledit agent rhéologique est l'alcool polyvinylique à 0,5 %.

12. Composition selon la revendication 3, où ladite composition contient en outre un tampon.

13. Composition selon la revendication 12, où ledit tampon est choisi dans le groupe constitué par les suivants : hydroxyde d'ammonium, carbonate, citrate, glycine, maléate, phosphates et leurs sels.

14. Composition selon la revendication 3, où ladite composition présente un pH compris entre 7 et 12.

15. Composition selon la revendication 14, où ladite composition présente un pH compris entre 7,5 et 9,5.

16. Composition selon la revendication 15, où ladite composition présente un pH de 8,5.

17. Composition selon la revendication 3, où ladite composition est stable au stockage.

18. Composition selon la revendication 3, où le pourcentage de particules de moins de 10 micromètres de diamètre dans le pulvérisateur est de 10 %.

19. Composition selon la revendication 18, où le pourcentage de particules de moins de 10 micromètres de diamètre dans le pulvérisateur est de 5 %.

20. Composition selon la revendication 3, où la granulométrie médiane dans le pulvérisateur est comprise entre 20 et 150 micromètres.

21. Composition selon la revendication 20, où la granulométrie médiane dans le pulvérisateur est comprise entre 40 et 100 micromètres.

22. Composition selon la revendication 21, où la granulométrie médiane dans le pulvérisateur est de 50 micromètres.

23. Composition selon la revendication 3, où ladite composition comprend en outre un composant actif supplémentaire choisi dans le groupe constitué par les suivants : analgésiques, anti-inflammatoires non stéroïdiens, corticoïdes, hyaluronane et opioïdes, y compris leurs sels.

24. Composition selon la revendication 23, où l'anti-inflammatoire est choisi dans le groupe constitué par les suivants : alosétron, anakinra, béclométhasone, bétaméthasone, budésonide, célécoxib, clobétasol, cromolyne, désoximétasone, dexaméthasone, épinastique, étanercept, étoricoxib, flunisolide, fluocinonide, fluticasone, formotérol, hydrocortisone, hydroxychloroquine, ibudilast, kétotifène, mésalamine, méthotrexate, méthylprednisolone, mométasone, montélukast, nédocromil, olsalazine, prednisone, ramatroban, rofécoxib, salbutamol, salmétérol, salsalate, terbutaline, triamcinolone, valdécoxib, zafirlukast, y compris leurs sels.

25. Composition selon la revendication 3, comprenant entre 0,1 et 2 % en masse de méloxicam, entre 1 et 10 mg/g d'alcool polyvinylique et entre 100 et 300 mg/g d'éthanol.

26. Composition pour utilisation dans le traitement d'un état pathologique chez un humain ou un animal non humain, où un volume de dose unitaire compris entre 25 microlitres et 400 microlitres de la composition pharmaceutique est pulvérisé sur la muqueuse orale de l'animal, où la composition pulvérisée présente une granulométrie médiane comprise entre 40 micromètres et 100 micromètres, et où l'agent pharmaceutique actif est absorbé au travers de la muqueuse orale de l'animal pour délivrer une quantité thérapeutiquement active de l'agent pharmaceutique actif au niveau du système circulatoire systémique pour soulager ledit état pathologique.

27. Composition pour utilisation selon la revendication 26, où la composition comprend du méloxicam, un alcool et un tampon.

28. Composition pour utilisation selon la revendication 26, où l'agent pharmaceutique actif est choisi dans le groupe constitué par les suivants : méloxicam, célécoxib, ondansétron, sumatriptan, zolpidem, tizanidine, ropinérole, insuline, glucose et nitroglycérine.

29. Composition pour utilisation selon la revendication 27, où la teneur en méloxicam est comprise entre 0,1 et 2 % en masse.

30. Composition pour utilisation selon la revendication 29, où la teneur en méloxicam est comprise entre 0,25 et 1 % en masse.

31. Composition pour utilisation selon la revendication 30, où la teneur en méloxicam est de 0,47 % en masse.

32. Composition pour utilisation selon la revendication 26, où le volume de pulvérisation est compris entre 50 et 400 microlitres.

33. Composition pour utilisation selon la revendication 32, où le volume de pulvérisation est compris entre 50 et 200 microlitres.

34. Composition pour utilisation selon la revendication 33, où le volume de pulvérisation est de 100 microlitres.

35. Composition pour utilisation selon la revendication 33, où le volume de pulvérisation est de 50 microlitres.

36. Composition pour utilisation selon la revendication 32, où le volume de pulvérisation est de 200 microlitres.

37. Composition pour utilisation selon la revendication 26, où le méloxicam est administré à une dose comprise entre 0,025 milligramme et 2 milligrammes par kilogramme par jour.

38. Composition pour utilisation selon la revendication 37, où le méloxicam est administré à une dose comprise entre 0,05 milligramme et 1 milligramme par kilogramme par jour.

39. Composition pour utilisation selon la revendication 38, où le méloxicam est administré à une dose comprise entre 0,1 milligramme et 0,2 milligramme par kilogramme par jour.

40. Composition pour utilisation selon la revendication 27, où l'état pathologique est choisi dans le groupe constitué par l'arthrose, la polyarthrite rhumatoïde, l'inflammation, la goutte et la douleur, et la composition comprend entre 0,1 et 2 % en masse de méloxicam, entre 1 et 10 mg/g d'alcool polyvinylique et entre 100 et 300 mg/g d'éthanol.
